# EUROPÄISCHE PATENTSCHRIFT

(11) **EP 1 004 564 B1**
(45) Veröffentlichungstag und Bekanntmachung des Hinweises auf die Patenterteilung: **16.04.2003**
(21) Anmeldenummer: 99121995.7
(22) Anmeldetag: 10.11.1999
(51) Int. Cl.: C07C 29/19, C07D 211/22

(54) **Verfahren zur Herstellung von Hydroxyethylcyclohexanen und Hydroxyethylpiperidinen**
Process for the preparation of hydroxyethylcyclohexanes and hydroxyethylpiperidines
Procédé pour la préparation d'hydroxyethylcyclohexanes et d'hydroxyethylpiperidines

(30) Priorität: 23.11.1998 DE 19853858
(43) Veröffentlichungstag der Anmeldung: 31.05.2000
(73) Patentinhaber: Bayer Aktiengesellschaft, 51368 Leverkusen (DE)
(72) Erfinder: Kiel, Wolfgang, Dr., 51519 Odenthal (DE)

(56) Entgegenhaltungen:
- DATABASE WPI Section Ch, Week 199205 Derwent Publications Ltd., London, GB; Class D23, AN 1992-037688 XP002134817 & JP 03 284640 A (SUMITOMO CHEM CO LTD), 16. Dezember 1991 (1991-12-16)
- PATENT ABSTRACTS OF JAPAN vol. 013, no. 362 (C-625), 14. August 1989 (1989-08-14) & JP 01 121226 A (SUMITOMO CHEM CO LTD), 12. Mai 1989 (1989-05-12)
- VASIL' EV A A ET AL: "Compounds with a herbal odor II. Cis-1-(2-Hydroxyethyl)-2-ethylcycloalkanes and their analogs" JOURNAL OF ORGANIC CHEMISTRY OF THE USSR. (ZHURNAL ORGANICHESKOI KHIMII), Bd. 27, Nr. 2, 1991, Seiten 273-278, XP002134815 CONSULTANTS BUREAU. NEW YORK., US
- BURTNER ROBERT R ET AL: "Antispasmodics III. Diarylacetic acid esters of some pyridyl and piperidyl alkanols" JOURNAL OF THE AMERICAN CHEMICAL SOCIETY., Bd. 69, 1947, Seiten 630-633, XP002134816 AMERICAN CHEMICAL SOCIETY, WASHINGTON, DC., US ISSN: 0002-7863
- DATABASE WPI Section Ch, Week 197706 Derwent Publications Ltd., London, GB; Class E13, AN 1977-006727 XP002134818 & RO 61 609 A (TERAPIA INTR MEDICA), 31. Juli 1976 (1976-07-31)

## Beschreibung

Die vorliegende Erfindung betrifft ein Verfahren zur Herstellung von Hydroxyethylcyclohexanen, die im Hexanring gegebenenfalls ein Stickstoffatom enthalten können, durch katalytische Hydrierung entsprechender Hydroxyethylbenzole bzw. Hydroxyethylpyridine.

Hydroxyethylcyclohexane und Hydroxyethylpiperidine sind Zwischenprodukte zur Herstellung von Pharmazeutika, Geruchsstoffen und Insekten-Repellants.

Database WPI section CH, AN 1992-037688 und Patent Abstracts of Japan Vol 013, NO 36266-625) beschreiben ein Verfahren zur Herstellung von Hydroxyethylcyclohexan durch Hydrierung von Hydroxyethylbenzol an einem geträgerten Rutheniumkatalysator, der durch Wasserstoff vorbehandelt wurde.

J. Org. Chem. USSR, 27(29), 1991, 273 beschreibt die Hydrierung von 1-(2-Hydroxyethyl)-2-ethylbenzol an einem Ruthenium/kohlerstoff-Katalysator, wobei als Lösungsmittel Isopropanol verwendet wird.

Wenn man Hydroxyethylcyclohexane und Hydroxyethylpiperidine mit üblichen Hydrierkatalysatoren wie Raney-Nickel oder Rhodium und/oder in polaren Lösungsmitteln durch Hydrierung der entsprechenden Hydroxyethylbenzole bzw. Hydroxyethylpyridine herstellt, finden in erheblichem Umfang Nebenreaktionen statt, die zur Bildung unerwünschter Nebenprodukte führen. Es besteht deshalb ein Bedürfnis nach einem Verfahren, mit dem sich Hydroxyethylcyclohexane, die im Cyclohexanring gegebenenfalls ein Stickstoffatom enthalten können, in guten Selektivitäten von beispielsweise über 95 % herstellen lassen.

Es wurde nun ein Verfahren zur Herstellung von Hydroxyethylcyclohexanen, die im Cyclohexanring gegebenenfalls ein Stickstoffatom enthalten können, durch katalytische Hydrierung der entsprechenden Hydroxyethylbenzole bzw. Hydroxyethylpyridine gefunden, das dadurch gekennzeichnet ist, daß man Ruthenium als Katalysator einsetzt, das vor dem Einsatz mit einem Reduktionsmittel behandelt wurde, und als Lösungsmittel ein Alkan verwendet, das bei Normaldruck einen Siedepunkt von über 70°C aufweist.

Erfindungsgemäß kann man beispielsweise Hydroxyethylbenzole und Hydroxyethylpyridine der Formel (I) einsetzen 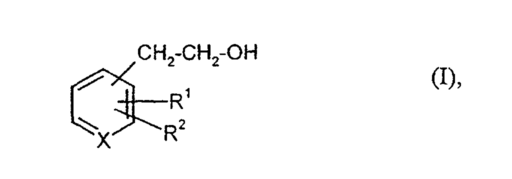 in der
- X: für CH oder N steht und
- R¹und R²: unabhängig voneinander jeweils Wasserstoff, Hydroxy, Amino, C₁-C₁₀-Alkyl, C₃-C₆-Cycloalkyl, C₆-C₁₀-Aryl, C₇-C₁₂-Aralkyl, C₁-C₁₀-Alkoxy oder C₃-C₆-Cycloalkoxy bedeuten,
und Hydroxyethylcyclohexane, die im Cyclohexanring gegebenenfalls ein Stickstoffatom enthalten können und der Formel (II) entsprechen 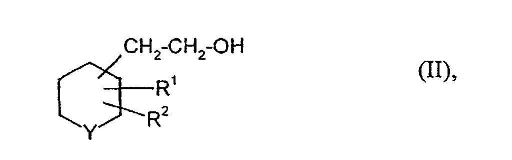 in der
- Y: für CH₂ oder NH steht und
- R¹und R²: die bei Formel (I) angegebene Bedeutung haben,
erhalten.

Vorzugsweise stehen in den Formeln (I) und (II) R¹ und R² unabhängig voneinander für Wasserstoff, C₁-C₄-Alkyl, Benzyl oder C₁-C₄-Alkoxy. Besonders bevorzugt stehen R¹ und R² für Wasserstoff.

In Formel (I) steht X vorzugsweise für N. Entsprechend steht in Formel (II) Y vorzugsweise für NH.

In den Formeln (I) und (II) liegt die Hydroxyethylgruppe vorzugsweise in der 2-Stellung in Bezug auf X bzw. Y vor. R¹ und R² befinden sich vorzugsweise in 3-, 4-, 5- und/oder 6-Stellung in Bezug auf X bzw. Y.

Besonders bevorzugt setzt man in das erfindungsgemäße Verfahren 2-(2-Hydroxyethyl)-pyridin oder Hydroxyethylbenzol ein und stellt 2-(2-Hydroxyethyl)-piperidin oder Hydroxyethylcyclohexan her.

Als Rutheniumkatalysatoren sind solche bevorzugt, die metallisches Ruthenium (d.h. Ruthenium der Oxidationsstufe ±0) auf einem Träger enthalten. Der Rutheniumgehalt solcher Trägerkatalysatoren kann z.B. 0,5 bis 15 Gew.-% betragen. Bevorzugt liegt er bei 1 bis 10 Gew.-%.

Die Behandlung des Rutheniums mit einem Reduktionsmittel vor dem Einsatz in das erfindungsgemäße Verfahren kann man beispielsweise mit Wasserstoff bei erhöhter Temperatur oder einem anderen geeigneten Reduktionsmittel, z.B. Hydrazin, durchführen. Wenn man mit Wasserstoff behandelt sind dabei z.B. Drucke von 120 bis 250 bar und Temperaturen von 120 bis 250°C anwendbar. Bevorzugt sind 150 bis 220 bar und 150 bis 220°C. Die Behandlung mit einem anderen geeigneten Reduktionsmittel, z.B. Hydrazin, kann man in Gegenwart eines Lösungsmittels bei Drucken von z.B. Normaldruck bis 5 bar und Temperaturen von z.B. 20 bis 80°C durchführen. Als Lösungsmittel kommen z.B. Wasser und organische Lösungsmittel in Frage wie Isopropanol und Methylcyclohexan sowie Säuren wie Schwefelsäure oder Essigsäure. Auch bereits hergestelltes Hydroxyethylcyclohexan, das im Cyclohexanring gegebenenfalls ein Stickstoffatom enthalten kann, kann als Lösungsmittel verwendet werden, wenn es unter den Behandlungsbedingungen flüssig ist. Die Behandlung mit Wasserstoff ist bevorzugt.

Bei dem Trägermaterial für die Trägerkatalysatoren kann es sich beispielsweise um Kohlen, Aluminiumoxide oder Kieselsäuren handeln. Es können auch andere bekannte Trägermaterialien für Metallkatalysatoren eingesetzt werden.

Für das erfindungsgemäße Verfahren gegebenenfalls nach einer Behandlung mit einem Reduktionsmittel geeignete Trägerkatalysatoren sind im Handel erhältlich.

Bei diskontinuierlicher Arbeitsweise kann man z.B. 0,01 bis 10 Gew.-% Ruthenium-Katalysator (nur Ru-Metall berücksichtigt und bezogen auf eingesetztes Hydroxyethylbenzol bzw. Hydroxyethylpyridin) einsetzen. Vorzugsweise liegt diese Menge bei 0,1 bis 2,5 Gew.-%.

Die Katalysatoren können nach Abtrennung aus dem Reaktionsgemisch erneut im erfindungsgemäßen Verfahren eingesetzt werden. Man kann das erfindungsgemäße Verfahren auch kontinuierlich durchführen.

Als Lösungsmittel werden Alkane verwendet, die bei Normaldruck einen Siedepunkt von über 70°C aufweisen, insbesondere geradkettige und verzweigte acyclische C₇-C₁₈-Alkane, unsubstituierte cyclische C₆-C₁₀-Alkane und mit geradkettigen oder verzweigten C₁-C₁₀-Alkylgruppen substituierte cyclische C₅-C₁₀-Alkane. Besonders bevorzugte Lösungsmittel sind Isooctan, Cyclohexan und Methylcyclohexan, insbesondere Methylcyclohexan. Man kann auch Gemische verschiedener Alkane einsetzen.

Bezogen auf 100 g eingesetztes Hydroxyethylbenzol bzw. Hydroxyethylpyridin kann man beispielsweise 10 bis 1000ml Lösungsmittel einsetzen. Vorzugsweise liegt diese Menge bei 20 bis 100 ml.

Die erfindungsgemäße Hydrierung kann beispielsweise bei Temperaturen im Bereich 50 bis 250°C und Wasserstoffdrucken von 5 bis 200 bar durchgeführt werden. Bevorzugt sind Temperaturen im Bereich 80 bis 220°C und Wasserstoffdrucke von 50 bis 180 bar.

Man kann das erfindungsgemäße Verfahren z.B. so durchführen, daß man zunächst das eingesetzte Hydroxyethylbenzol bzw. Hydroxyethylpyridin gegebenenfalls zusammen mit dem Lösungsmittel in einem Druckgefäß vorlegt und das Druckgefäß z.B. durch Spülen mit Stickstoff inertisiert. Der Katalysator ist, vorzugsweise separat davon, mit Wasserstoff oder einem anderen geeigneten Reduktionsmittel vorbehandelt worden. Den Katalysator gibt man, bevorzugt suspendiert in dem gleichen Lösungsmittel wie bereits im Druckgefäß befindlich, in das Druckgefäß, das man anschließend auf die Reaktionstemperatur erhitzt und Wasserstoff aufdrückt. Die Reaktion ist i.a. nach 30 Minuten bis 10 Stunden beendet (keine Wasserstoffaufnahme mehr). Man kann dann das vorliegende Gemisch aufarbeiten, indem man es zunächst abkühlt, den Druck entspannt, dann den Katalysator abtrennt und das hergestellte Hydroxyethylcyclohexan bzw. Hydroxyethylpiperidin z.B. durch Destillation isoliert.

Es sind auch andere Ausführungsformen des erfindungsgemäßen Verfahrens möglich, insbesondere auch kontinuierlich durchzuführende.

Mit dem erfindungsgemäßen Verfahren gelingt es Hydroxyethylcyclohexane und Hydroxyethylpiperidine in hohen Ausbeuten und mit sehr hohen Selektivitäten herzustellen. Die Selektivitäten, bezogen auf umgesetztes Ausgangsmaterial, liegen i.a. bei über 95 %. häufig bei über 98 %. Außerdem sind beim erfindunggemäßen Verfahren die Standzeiten der Katalysatoren hoch.

### Beispiele

### Beispiel 1

150 g 2-(2-Hydroxyethyl)-pyridin wurden mit 50 g Methylcyclohexan vermischt und in einem 0,71 Rührautoklaven gegeben. Der Autoklav wurde dann 3 mal mit 5 bar N₂ inertisiert. Anschließend wurden 10 eines 5 Gew.-% Ruthenium auf Kohle enthaltenden Katalysators, suspendiert in 50 ml Methylcyclohexan zugepumpt. Der Katalysator war zuvor bei 200°C und 200 bar mit H₂ behandelt worden. Der Ansatz wurde bei 150°C und 80 bar H₂-Druck hydriert. Die Hydrierzeit betrug 5 Stunden.

Es wurde ein Rohgemisch folgender Zusammensetzung (GC) erhalten:

| | |
|---|---|
| 2-(2-Hydroxyethyl)-piperidin | 98,6 % |
| 2-Ethylpiperidin | 0,4 % |
| sonstige Ethylpiperidine | 0,5 % |

### Beispiel 2

Es wurde gearbeitet wie in Beispiel 1, jedoch wurden 10g eines Katalysators verwendet, der 5 Gew.-% Ruthenium auf Aluminiumoxid enthielt. Das erhaltene Rohgemisch enthielt 96,6 % (GC) 2-(2-Hydroxyethyl)-piperidin.

### Beispiel 3 (zum Vergleich)

Es wurde gearbeitet wie in Beispiel 1, jedoch wurden 3 g Raney-Nickel als Katalysator eingesetzt. Das erhaltene Rohgemisch enthielt (GC):

| | |
|---|---|
| 2-(2-Hydroxyethyl)-piperidin | 48,6 % |
| Methylpiperidine | 19,9 % |
| Hydroxyethyl-ethylpiperidine | 19,3 % |

### Beispiel 4 (zum Vergleich)

Es wurde gearbeitet wie in Beispiel 1, jedoch wurden 10 g eines 5 Gew.-% Rhodium auf Kohle enthaltenden Katalysators eingesetzt. Das erhaltene Rohgemisch enthielt (GC):

| | |
|---|---|
| 2-(2-Hydroxyethyl)-piperidin | 79,7 % |
| Hydroxyethylpyridin | 14,9 % |
| Ethylpiperidine | 3,8 % |

### Beispiel 5

150 g Hydroxyethylbenzol wurden mit 50 g Methylcyclohexan vermischt un in einen 0,7 1 Rührautoklaven gegeben. Der Autoklav wurde dann 3 mal mit 5 bar N₂ inertisiert. Anschließend wurden 5 g eines 5 Gew.-% Ruthenium auf Kohle enthaltenden Katalysators, suspendiert in 50 ml Methylcyclohexan zugepumpt. Der Katalysator war zuvor bei 200°C und 200 bar mit H₂ behandelt worden. Der Ansatz wurde bei 100°C und 150 bar H₂-Druck hydriert. Die Hydrierzeit betrug 45 Minuten.

Es wurde ein Rohgemisch folgender Zusammensetzung (GC) erhalten:

| | |
|---|---|
| Hydroxyethylcyclohexan | 99,3 % |
| Ethylcyclohexan | 0,3 % |

### Beispiel 6 (zum Vergleich)

Es wurde gearbeitet wie in Beispiel 1, jedoch wurden statt je 50 g Methylcyclohexan je 100 g Methanol eingesetzt. Das erhaltene Rohgemisch enthielt (GC):

| | |
|---|---|
| 2-(2-Hydroxyethyl)-piperidin | 81,5 % |
| Methylpiperidin | 6,3 % |
| Ethylpiperidin | 4,4 % |
| Hydroxyethyl-ethylpiperidin | 6,6 % |
| Unbekannte Substanzen | Rest |

## Patentansprüche

1. Verfahren zur Herstellung von Hydroxyethylcyclohexanen, die im Cyclohexanring gegebenenfalls ein Stickstoffatom enthalten können, durch katalytische Hydrierung der entsprechenden Hydroxyethylbenzole bzw. Hydroxyethylpyridine, **dadurch gekennzeichnet, dass** man Ruthenium als Katalysator einsetzt, das vor dem Einsatz mit einem Reduktionsmittel behandelt wurde und als Lösungsmittel ein Alkan verwendet, das bei Normaldruck einen Siedepunkt von über 70°C aufweist.

2. Verfahren nach Anspruch 1, **dadurch gekennzeichnet, dass** man Hydroxyethylbenzole oder Hydroxyethylpyridine der Formel (I) einsetzt 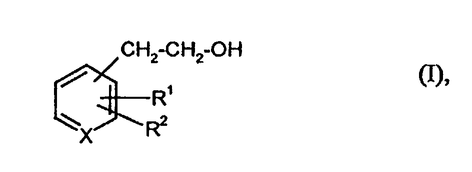 in der
X für CH oder N steht und
R¹ und R² unabhängig voneinander jeweils Wasserstoff, Hydroxy, Amino, C₁-C₁₀-Alkyl, C₃-C₆-Cycloalkyl, C₆-C₁₀-Aryl, C₇-C₁₂-Aralkyl, C₁-C₁₀-Alkoxy oder C₃-C₆-Cycloalkoxy bedeuten,
und Hydroxyethylcyclohexane, die im Cyclohexanring gegebenenfalls ein Stickstoffatom enthalten können und der Formel (II) entsprechen 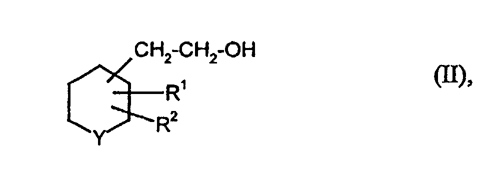 in der
Y für CH₂ oder NH steht und
R¹ und R² die bei Formel (I) angegebene Bedeutung haben,
erhält.

3. Verfahren nach Ansprüchen 1 bis 2, **dadurch gekennzeichnet, dass** man als Ruthenium-Katalysatoren solche einsetzt, die metallisches Ruthenium auf einem Träger enthalten.

4. Verfahren nach Ansprüchen 1 bis 3, **dadurch gekennzeichnet, dass** man Ruthenium enthaltende Trägerkatalysatoren einsetzt, die vor ihrem Einsatz bei erhöhter Temperatur mit Wasserstoff oder einem anderen Reduktionsmittel behandelt wurden.

5. Verfahren nach Ansprüchen 1 bis 4, **dadurch gekennzeichnet, dass** man die Vorbehandlung des Rutheniums mit Wasserstoff bei Temperaturen von 120 bis 250°C und Drucken von 120 bis 250 bar durchführt.

6. Verfahren nach Ansprüchen 1 bis 5, **dadurch gekennzeichnet, dass** man Ruthenium enthaltende Trägerkatalysatoren einsetzt, die als Trägermaterial Kohlen, Aluminiumoxide oder Kieselsäuren enthalten.

7. Verfahren nach Ansprüchen 1 bis 6, **dadurch gekennzeichnet, dass** man bei kontinuierlicher Arbeitsweise 0,01 bis 10 Gew.-% Rutheniumkatalysator (nur Ru-Metall) berücksichtigt, bezogen auf eingesetztes Hydroxyethylbenzol bzw. Hydroxyethylpyridin, einsetzt.

8. Verfahren nach Ansprüchen 1 bis 7, **dadurch gekennzeichnet, dass** man als Alkane Isooctan, Cyclohexan oder Methylcyclohexan verwendet.

9. Verfahren nach Ansprüchen 1 bis 8, **dadurch gekennzeichnet, dass** man die Hydrierung bei Temperaturen im Bereich 50 bis 250°C und Wasserstoffdrucken im Bereich 5 bis 200 bar durchführt.

## Claims

1. Process for the preparation of hydroxyethylcyclohexanes which can optionally contain a nitrogen atom in the cyclohexane ring by catalytic hydrogenation of the corresponding hydroxyethylbenzenes or hydroxyethylpyridines, **characterized in that** the catalyst used is ruthenium which, prior to use, has been treated with a reducing agent, and the solvent used is an alkane which has a boiling point above 70°C at atmospheric pressure.

2. Process according to Claim 1, **characterized in that** hydroxyethylbenzenes or hydroxyethylpyridines of the formula (I) 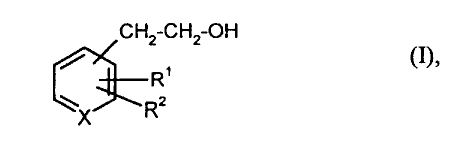 in which
X is CH or N and
R¹ and R² independently of one another are each hydrogen, hydroxyl, amino, C₁-C₁₀-alkyl, C₃-C₆-cycloalkyl, C₆-C₁₀-aryl, C₇-C₁₂-aralkyl, C ₁-C₁₀-alkoxy or C₃-C₆-cycloalkoxy, are used
and hydroxyethylcyclohexanes which can optionally contain a nitrogen atom in the cyclohexane ring and correspond to the formula (II) 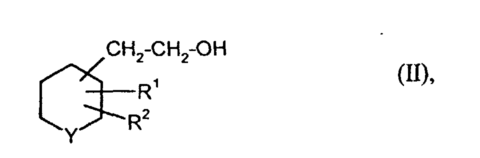 in which
Y is CH₂ or NH and
R¹ and R² are as defined for formula (I),
are obtained.

3. Process according to Claims 1 and 2, **characterized in that** the ruthenium catalysts used are those which comprise metallic ruthenium on a support.

4. Process according to Claims 1 to 3, **characterized in that** ruthenium-containing supported catalysts are used which, prior to their use, have been treated with hydrogen or another reducing agent at elevated temperatures.

5. Process according to Claims 1 to 4, **characterized in that** the pretreatment of the ruthenium with hydrogen is carried out at temperatures of from 120 to 250°C and pressures of from 120 to 250 bar.

6. Process according to Claims 1 to 5, **characterized in that** ruthenium-containing supported catalysts which contain, as support material, charcoals, aluminium oxides or silicas are used.

7. Process according to Claims 1 to 6, **characterized in that** in the continuous procedure from 0.01 to 10% by weight of ruthenium catalyst (taking only Ru metal into account), based on hydroxyethylbenzene or hydroxyethylpyridine used, is used.

8. Process according to Claims 1 to 7, **characterized in that** the alkanes used are isooctane, cyclohexane or methylcyclohexane.

9. Process according to Claims 1 to 8, **characterized in that** the hydrogenation is carried out at temperatures in the range from 50 to 250°C and hydrogen pressures in the range from 5 to 200 bar.

## Revendications

1. Procédé pour la préparation d'hydroxyéthylcyclohexanes, lesquels peuvent éventuellement contenir un atome d'azote dans le cycle cyclohexane, par hydrogénation catalytique des hydroxyéthylbenzènes respectivement hydroxyéthylpyridines correspondants, **caractérisé en ce que** l'on utilise comme catalyseur du ruthénium, lequel a été traité avant l'utilisation avec un agent réducteur et on utilise comme solvant un alcane, lequel présente à pression atmosphérique un point d'ébullition supérieur à 70°C.

2. Procédé selon la revendication 1, **caractérisé en ce que** l'on utilise des hydroxyéthylbenzènes ou hydroxyéthylpyridines de la formule (I) 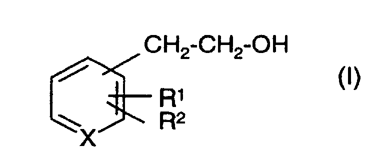 dans laquelle
X représente CH ou N et
R¹ et R² représentent indépendamment à chaque fois un atome d'hydrogène, un groupe hydroxy, amino, alkyle en C₁-C₁₀, cycloalkyle en C₃-C₆, aryle en C₆-C₁₀, aralkyle en C₇-C₁₂, alcoxy en C₁-C₁₀ ou cycloalcoxy en C₃-C₆,
et on obtient des hydroxyéthylcyclohexanes, lesquels peuvent éventuellement contenir un atome d'azote dans le cycle cyclohexane et qui correspondent à la formule (II) 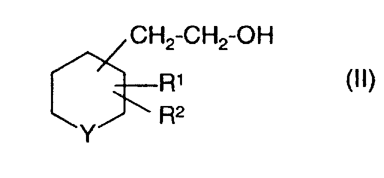 dans laquelle
Y représente CH₂ ou NH et
R¹ et R² ont la signification indiquée dans la formule (I).

3. Procédé selon les revendications 1 à 2, **caractérisé en ce que** l'on utilise comme catalyseurs de ruthénium ceux qui contiennent du ruthénium métallique sur un support.

4. Procédé selon les revendications 1 à 3, **caractérisé en ce que** l'on utilise des catalyseurs sur support contenant du ruthénium, lesquels ont été traités avant leur utilisation à une température élevée avec de l'hydrogène ou avec un autre agent réducteur.

5. Procédé selon les revendications 1 à 4, **caractérisé en ce que** l'on réalise le prétraitement du ruthénium avec de l'hydrogène à des températures de 120 à 250°C et à des pressions de 120 à 250 bar.

6. Procédé selon les revendications 1 à 5, **caractérisé en ce que** l'on utilise des catalyseurs sur support contenant du ruthénium, lesquels contiennent comme matériau de support du carbone, de l'oxyde d'aluminium ou des acides siliciques.

7. Procédé selon les revendications 1 à 6, **caractérisé en ce que** l'on utilise pour une procédure continue de 0,01 à 10 % en poids de catalyseur de ruthénium (uniquement métal Ru), rapportés à l'hydroxyéthylbenzène respectivement l'hydroxyéthylpyridine utilisé.

8. Procédé selon les revendications 1 à 7, **caractérisé en ce que** l'on utilise comme alcane de l'isooctane, du cyclohexane ou du méthylcyclohexane.

9. Procédé selon les revendications 1 à 8, **caractérisé en ce que** l'on réalise l'hydrogénation à des températures dans un domaine de 50 à 250°C et à des pressions d'hydrogène dans le domaine de 5 à 200 bar.
